# EUROPEAN PATENT APPLICATION

(11) **EP 2 506 587 A2**
(43) Date of publication of application: **03.10.2012**
(21) Application number: 12157127.7
(22) Date of filing: 27.02.2012
(51) Int. Cl.: H04N 13/04, A61B 6/02

(54) **Stereoscopic display apparatus**

(30) Priority: 31.03.2011 JP 2011079387
(71) Applicant: Fujifilm Corporation, Minato-ku Tokyo (JP)
(72) Inventor: Kanagawa, Akiko, Kanagawa (JP); Kaneko, Yasuhiko, Kanagawa (JP); Hiruoka, Jun, Kanagawa (JP); Sakaguchi, Yuichi, Kanagawa (JP); Mochizuki, Naoki, Kanagawa (JP); Ida, Noriaki, Kanagawa (JP)
(74) Representative: Klunker . Schmitt-Nilson . Hirsch

(57) **Abstract**

A stereoscopic display apparatus that can perform a stereoscopic display using a right-eye image and a left-eye image acquired through radiography, includes image display means that can display a planar image of at least one of the right-eye image and the left-eye image as a planar display along with the stereoscopic display, display selecting means that selects any one of the stereoscopic display and the planar display, and position pointing means that can be operated in the selected stereoscopic display or planar display and that can be operated to interlock in the stereoscopic display and the planar display, wherein the position pointing means includes depth changing means that changes the depth thereof in the stereoscopic display.

## Description

### BACKGROUND OF THE INVENTION

This invention relates to a stereoscopic display apparatus that performs a stereoscopic display using a right-eye image and a left-eye image acquired through radiography.

Recently, medical image-related network systems such as PACS (Picture Archiving and Communication Systems) have been developed and opportunities for storing radiographic images during diagnosis as digital data in a server and using the digital data for follow-up later or transmitting the radiographic images during the previous diagnosis as image data for use in other remote hospitals increase.

In radiography, the invention of a stereoscopic display apparatus of a radiographic image that irradiates a subject with radiation from different angles, that acquires a right-eye image and a left-eye image, and that performs a stereoscopic display using the acquired images is known.

In fluoroscopic images such as radiographic images, it is difficult to determine which part of an image is located in the foreground and to discern in the depth direction. Therefore, when a radiographic image is stereoscopically displayed, there is a merit that it is easy to determine the three-dimensional distribution of pathological changes or the like (see JP 2010-200787 A and JP 3780217 B).

### SUMMARY OF THE INVENTION

In designation of a position for biopsy (tissue extraction), display of a CT image, or the like, techniques of calculating positional relations between plural images and shifting position designating means such as a marker in an image to interlock a marker indicating the same position in another image therewith are known. These techniques project 3D data to a plane and are different from a stereoscopic display allowing a human being to recognize two different images as a stereoscopic image using human perception.

Particularly, in the case of a 3D projection image, a displayed image is the same to anyone. However, a stereoscopic image is based on an observer's recognition and the stereoscopic image itself does not exist. Accordingly, since it is difficult to explain a stereoscopic image to a third party and a stereoscopic image cannot be observed without using an apparatus coping with the stereoscopic image, it is necessary to store information suitable for a display of a 2D image (planar image), not the stereoscopic image. Therefore, an observer preferably points a position or the like while checking the planar display along with the stereoscopic display.

An object of the present invention is to provide a stereoscopic display apparatus performing a stereoscopic display using a right-eye image and a left-eye image acquired through radiography, which can perform a planar display of at least one of the right-eye image and the left-eye image at the same time as the stereoscopic display and which includes position pointing means coupling the stereoscopic display and the planar display to allow an observer to confirm the positions of the position pointing means in both the stereoscopic display and the planar display.

In order to achieve the above-mentioned objects, the resent invention provides a stereoscopic display apparatus that can perform a stereoscopic display using a right-eye image and a left-eye image acquired through radiography, comprising:
image display means that can display a planar image of at least one of the right-eye image and the left-eye image as a planar display along with the stereoscopic display;
display selecting means that selects any one of the stereoscopic display and the planar display; and
position pointing means that can be operated in the selected stereoscopic display or planar display and that can be operated to interlock in the stereoscopic display and the planar display,
wherein the position pointing means includes depth changing means that changes the depth thereof in the stereoscopic display.

Further, preferably, when the depth of the position pointing means is changed, the position of the position pointing means in at least one of the right-eye display and the left-eye display is changed with the change of the depth.

Further, preferably, when the position of the position pointing means in at least one of the right-eye display and the left-eye display is changed, the depth of the position pointing means in the stereoscopic display is changed.

Further, preferably, the position pointing means includes position designating means that can designate a position when disposed in the selected stereoscopic display or planar display and that is disposed to interlock in the stereoscopic display and the planar display,
wherein the depth changing means can change the depth of the position designating means.

Further, preferably, when the depth of the position designating means is changed, the position of the position designating means in at least one of the right-eye display and the left-eye display is changed with the change of the depth.

Further, preferably, when the arrangement of the position designating means in at least one of the right-eye display and the left-eye display is changed, the depth of the position designating means in the stereoscopic display is changed.

Further, preferably, a plurality of the position designating means can be arranged.

Further comprising warning means that gives a warning when the position pointing means for a left eye and the position pointing means for a right eye in the stereoscopic display are separated from each other by a predetermined distance or more.

Further, preferably, the warning includes stopping the changing of the distance between the position pointing means for the left eye and the position pointing means for the right eye, re-interlocking of the position pointing means for the left eye and the position pointing means for the right eye with each other, and displaying the purports of the stopping and the re-interlocking.

Further, preferably, the stereoscopic display and at least one of the right-eye display and the left-eye display are displayed on the single image display means.

Further, preferably, a plurality of the image display means are provided, and
wherein the stereoscopic display and at least one of the right-eye display and the left-eye display are displayed on the different image display means, respectively.

According to the present invention, in radiographic image diagnosis in which discernment is difficult in the depth direction because it is a transmission image, it is possible to simply point a position in the depth direction in a stereoscopic display and to confirm the pointed position in the depth direction in a planar display, thereby enhancing precision in radiographic image diagnosis.

Since no image process is performed on a captured image for the stereoscopic display and the pointing of a position in the depth direction, it is possible to perform radiographic image diagnosis on the basis of accurate captured images.

### BRIEF DESCRIPTION OF THE DRAWINGS

FIG. 1 is an overview diagram illustrating an example of a stereoscopic display apparatus according to the present invention.
FIG. 2 is a block diagram illustrating an example of the system configuration of the stereoscopic display apparatus according to the present invention.
FIG. 3A is a diagram illustrating a stereoscopic display of the stereoscopic display apparatus according to the present invention, FIG. 3B is a diagram illustrating a left-eye display thereof, and FIG. 3C is a diagram illustrating a right-eye display thereof.
FIG. 4 is a diagram illustrating the operation of a display selecting means (selection cursor) of the stereoscopic display apparatus according to the present invention.
FIG. 5 is a diagram illustrating the operation of position pointing means (pointing cursor) of the stereoscopic display apparatus according to the present invention.
FIG. 6 is a diagram illustrating the operation of position designating means (marker) of the stereoscopic display apparatus according to the present invention.
FIG. 7 is a diagram illustrating the adjustment of the position designating means (adjustment in a left-eye display) of the stereoscopic display apparatus according to the present invention.
FIG. 8 is a diagram illustrating the adjustment of the position designating means (adjustment in a right-eye display) of the stereoscopic display apparatus according to the present invention.
FIG. 9 is a flowchart illustrating the flow of processes when arranging the position designating means in a stereoscopic display.
FIG. 10 is a flowchart illustrating the flow of processes when finely adjusting the depth of the position designating means in a stereoscopic display.

### DETAILED DESCRIPTION OF THE INVENTION

A radiographic-image stereoscopic display apparatus according to the present invention will be described below in detail with reference to exemplary embodiments shown in the accompanying drawings.

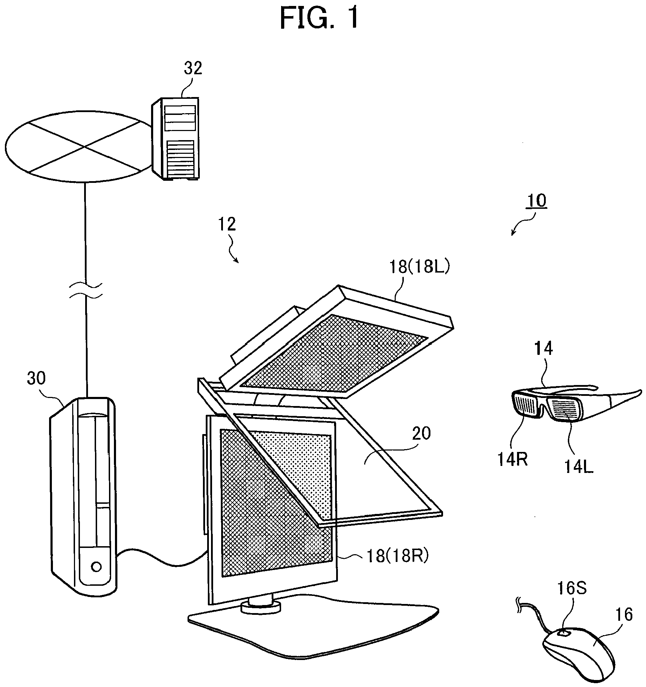

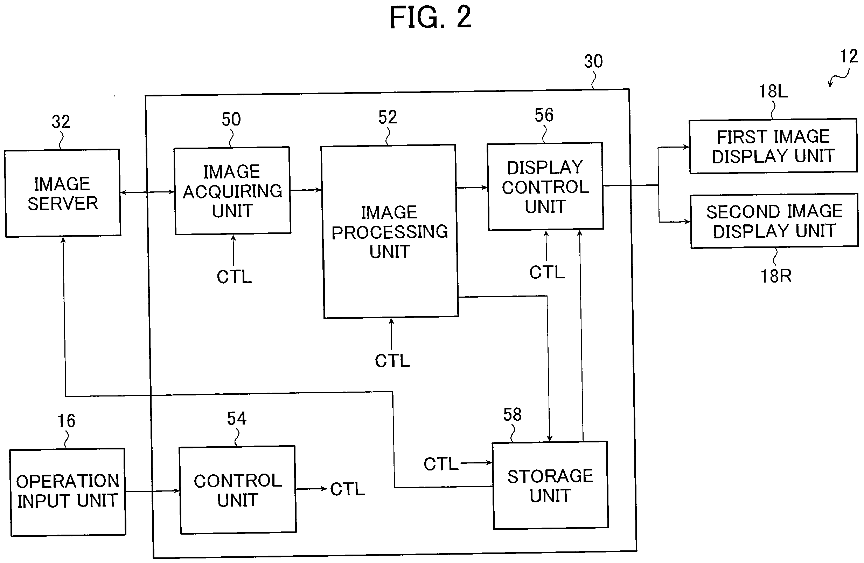

FIG. 1 is an overview diagram illustrating the entire configuration of an example of a stereoscopic display apparatus according to the present invention. FIG. 2 is an example of a block diagram illustrating the system configuration of the stereoscopic display apparatus according to the present invention.

The stereoscopic display apparatus 10 according to the present invention includes a stereoscopic display unit (image display means) 12, a pair of polarizing glasses 14, an operation input unit 16, and a console 30.

The stereoscopic display unit (image display means) 12 includes a first image display unit 18L, a second image display unit 18R, and a beam splitter mirror 20. The stereoscopic display unit 12 performs a stereoscopic display by displaying a left-eye image on the first image display unit 18L and displaying a right-eye image on the second image display unit 18R, and may also display a normal planar display by displaying the same image on the first image display unit 18L and the second image display unit 18R.

It is assumed that the stereoscopic display unit 12 is observed from an observation position apart by a predetermined distance from the stereoscopic display unit 12 (predetermined observation conditions) and that an inter-pixel distance (pixel size) of the stereoscopic display unit 12 (the first image display unit 18L and the second image display unit 18R) and an enlargement and reduction ratio are set to predetermined values (predetermined display conditions and image processing conditions).

By causing an observer to observe the stereoscopic display unit 12 from the above-mentioned observation position by the use of a pair of polarizing glasses 14 to be described later, it is possible to achieve a stereoscopic display based on a right-eye image and a left-eye image.

The stereoscopic display apparatus 10 according to the present invention is connected, for example, to an image network system such as PACS via an image acquiring unit 50 to be described later of the console 30 as described above and acquires a right-eye image and a left-eye image allowing a stereoscopic view in a radiographic image from an image server 32 included in the image network system. The stereoscopic display apparatus 10 may be connected directly to a radiographic imaging system or the like and may acquire a right-eye image and a left-eye image allowing a stereoscopic view.

The right-eye image and the left-eye image allowing a stereoscopic view are a pair of radiographic images radiographed (stereoscopically photographed) under predetermined radiographic conditions in a state where the irradiation position of radiation, that is, a bulb (radiation source) position, is shifted in a predetermined direction by a predetermined distance in a radiographic imaging apparatus or the like.

The right-eye image and the left-eye image allowing a stereoscopic view may be a pair of radiographic images radiographed in a state where the irradiation direction of the bulb is shifted or may be a pair of radiographic images radiographed in a state where a subject rotates and an incidence angle of radiation on a subject is changed.

The acquired right-eye image and left-eye image are subjected to a predetermined image process by an image processing unit 52 to be described later of the console 30 and are displayed on the first image display unit 18L and the second image display unit 18R, respectively, by a display control unit 56 to be described later.

Optical filters which are not shown in the drawings and which polarize light emitted from the display units in predetermined different directions may be disposed in front of the first image display unit 18L and the second image display unit 18R.

Here, the first light of the left-eye image displayed on the first image display unit 18L is polarized in a predetermined direction by the optical filter not shown and is reflected by the beam splitter mirror 20.

Similarly, the second light of the right-eye image displayed on the second image display unit 18R is polarized in a predetermined direction different from that of the first light by the optical filter not shown and is transmitted by the beam splitter mirror 20.

Therefore, the reflected first light and the transmitted second light are changed to combined light having different polarization directions and travelling in the same direction by the beam splitter mirror 20.

When the light displayed on the first image display unit 18L is reflected by the beam splitter mirror 20, the first light is vertically inverted (exactly, the front and rear sides in the travelling direction of the light are inverted), and it is thus necessary to vertically invert a display image in advance. Therefore, the image processing unit 52 to be described later of the console 30 performs a process of vertically inverting an image on the left-eye image displayed on the first image display unit 18L in advance, in addition to a process of changing an enlargement and reduction ratio of both images for display.

The angle of the beam splitter mirror 20 is adjusted and fixed so that the left-eye image displayed on the first image display unit 18L and the right-eye image displayed on the second image display unit 18R overlap with each other when an operator (an observer) at the observation position views the display unit 12 of the stereoscopic display apparatus 10 from the front side.

The pair of polarizing glasses 14 transmits the first light from the first image display unit 18L through the use of a left-eye polarizing lens 14L, transmits the second light from the second image display unit 18R through the use of a right-eye polarizing lens 14R, and blocks the other light.

Therefore, the left-eye image displayed on the first image display unit 18L is recognized by the left eye of the operator wearing the pair of polarizing glasses 14 and the right-eye image displayed on the second image display unit 18R is recognized by the right eye of the operator.

When recognizing images having parallax by the sue of the left eye and the right eye, a human being recognizes the image as a stereoscopic display. Accordingly, the operator wearing the pair of polarizing glasses 14 observes a stereoscopic display based on the right-eye and the left-eye image by simultaneously recognizing the right-eye image and the left-eye image having parallax by the sue of the right eye and the left eye.

The operation input unit 16 may be, for example, a pointing device such as a mouse used for operating a computer and display selecting means (selection cursor) and position pointing means (pointing cursor) to be described later on the display screen recognized by the operator can be freely operated by operating the operation input unit 16. The operation input unit 16 is not limited to these, but may further include operation input means such as a keyboard for inputting information in addition to the mouse.

Although details will be described later, the positions of these cursors are calculated and the cursors are drawn by the use of the console 30 and are displayed on the display screen.

As shown in FIG. 2, the console 30 includes an image acquiring unit 50, an image processing unit 52, a control unit 54, a display control unit 56, and a storage unit 58. The console 30 is specifically constructed by a computer including a CPU (Central Processing Unit), a RAM (Random Access Memory), and a hard disk and the CPU, the RAM, the hard disk, and the like constitute the units of the console 30 in cooperation with each other.

The image acquiring unit 50 acquires image data of a right-eye image and a left-eye image allowing a stereoscopic display from the image server 32 in response to the operation input unit 16 via the control unit 54.

The image processing unit 52 performs a predetermined image process on the image data of the right-eye image and the left-eye image acquired by the image acquiring unit 50 and outputs image data which can be displayed by the first image display unit 18L and the second image display unit 18R. The image processing unit 52 performs an enlargement and reduction process using a predetermined enlargement and reduction ratio on the right-eye image and the left-eye image on the basis of display software or the like. The enlargement and reduction process is a process of displaying both the right-eye image and the left-eye image on the stereoscopic display unit 12 (the first image display unit 18L and the second image display unit 18R). As described above, an image process is performed on the left-eye image to be displayed on the first image display unit 18L so as to display a vertically-inverted image.

The image processing unit 52 may output the image data of the right-eye image and the left-eye image having been subjected to the image process to the storage unit 58 in response to an instruction from the control unit 54.

The control unit 54 controls the operations of the image acquiring unit 50, the image processing unit 52, the display control unit 56, and the storage unit 58 in accordance with an instruction from the operation input unit 16 and displays the cursors (the selection cursors and the pointing cursors) operated by the operation input unit 16 on the display screen 20 through the use of the display control unit 56.

The control unit 54 controls image selection by the selection cursor to be described later in response to an instruction from the operation input unit 16, calculates a variation in position of the pointing cursor in the right-eye image and the left-eye image and a variation in depth of the pointing cursor in the stereoscopic display, and displays the pointing cursor in both images.

The display control unit 56 displays the right-eye image and the left-eye image, which has been subjected to a predetermined image process by the image processing unit 52 and can be displayed, on the first image display unit 18L and the second image display unit 18R in response to an instruction from the control unit 54, and the control unit 54 displays the cursor 24, which is calculated in position and drawn, on the first image display unit 18L and the second image display unit 18R.

The display control unit 56 may read the image data of the right-eye image and the left-eye image stored in the storage unit 58 in response to an instruction from the control unit 54 and may display the image data on the first image display unit 18L and the second image display unit 18R.

The storage unit 58 stores the image data of the right-eye image and the left-eye image having been subjected to the predetermined image process by the image processing unit 52 and outputs the image data to the display control unit 56, if necessary, in response to the instruction from the control unit 54.

The storage unit 58 may output the image data of the stored right-eye image and left-eye image to the image server 32 in response to an instruction from the operation input unit 16 via the control unit 54.

The storage unit 58 may store marker positions to be described later, and the like, in addition to the image data of the right-eye image and the left-eye image.

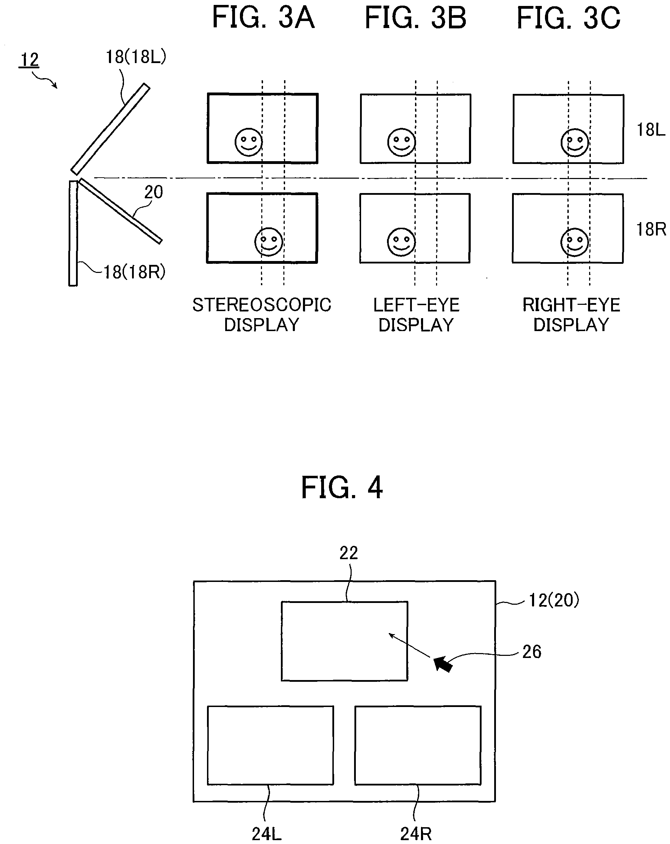

FIGS. 3A, 3B, and 3C are examples of a screen display recognized in the display unit 12 (the beam splitter mirror 20) of the stereoscopic display apparatus 10 according to the present invention. FIG. 3A shows a stereoscopic display, FIG. 3B shows a left-eye display, and FIG. 3C shows a right-eye display.

The upper images in FIGS. 3A to 3C are images displayed on the first image display unit 18L and the lower images in FIGS. 3A to 3C are images displayed on the second image display unit 18R.

In the stereoscopic display shown in FIG. 3A, the display positions of the upper image and the lower image are shifted in a predetermined direction. Accordingly, an operator can recognize the combined image as a stereoscopic display through the use of a pair of polarizing glasses 14. In the left-eye image shown in FIG. 3B and the right-eye image shown in FIG. 3C, the display positions of the upper image and the lower image are matched with each other. Accordingly, the operator recognizes the right-eye image and the left-eye image as a planar display. In the stereoscopic display shown in FIG. 3A, the larger the distance between the right-eye image and the left-eye image is, the farther the combined image appears. In addition, when the positions of the right-eye image and the left-eye image are exchanged, the combined image appears popped up.

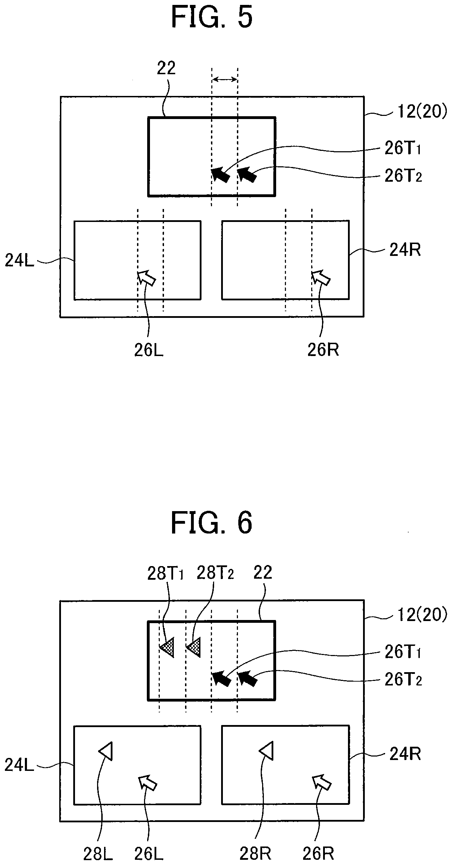

FIGS. 4 and 5 show a display screen of the stereoscopic display unit 12 (the beam splitter mirror 20) recognized by the operator. In FIGS. 4 and 5, a stereoscopic display 22, a left-eye display 24L, and a right-eye display 24R are displayed on the same screen. The present invention is not limited to this configuration, but display devices corresponding to the stereoscopic display 22, the left-eye display 24L, and the right-eye display 24R, respectively may be provided instead of displaying the displays on the same screen.

The display selecting means (selection cursor) 26 shown in FIG. 4 selects any one of the stereoscopic display 22, the left-eye display 24L, and the right-eye display 24R on the display screen of the stereoscopic display unit 12.

For example, any display is selected by superimposing the selection cursor 26 on any of the displays and selecting the superimposed display through the use of the operation input unit 16 (for example, clicking the superimposed display through the use of the mouse 16).

When any display is selected, the selection cursor 26 is changed to any of position pointing means (pointing cursors) 26T₁, 26T₂, 26L, and 26R depending on the selected display.

FIGS. 4 to 6 are diagrams illustrating a case where the stereoscopic display 22 is selected with the selection cursor 26. The change between the selection cursor 26 and the pointing cursors 26T₁, 26T₂, 26L, and 26R can be performed at any time through the use of predetermined means. For example, the pointing cursor may be changed to the selection cursor by clicking the right side of the mouse and selecting a displayed menu.

When a display to be operated is selected with the selection cursor 26, the pointing cursor is also displayed in the display (the left-eye display 24L and the right-eye display 24R when the stereoscopic display 22 is selected) other than the selected display by interlocking with the pointing cursor in the selected display. When the pointing cursor is changed to the selection cursor, the pointing cursor displayed in the display other than the selected display disappears.

FIG. 5 is a diagram illustrating a case where the stereoscopic display 22 is designated in FIG. 4. The operator views the stereoscopic display through the use of the pair of polarizing glasses 14, but both the right-eye display and the left-eye display are displayed in the display when the pair of polarizing glasses 14 is removed, that is, in the stereoscopic display 22, for the purpose of convenient explanation. The pointing cursor 26L moves in the left-eye display 24L and the pointing cursor 26R moves in the right-eye display 24R by interlocking with the movement of the pointing cursors 26T₁ and 26T₂ in the stereoscopic display 22.

As shown in FIG. 5, when the display to be operated is selected by the use of the display selecting means, the periphery of the display is emphasized. The color of the pointing cursor may be emphasized (for example, by raising the chroma) and the pointing cursor in the display not selected may be outlined. The pointing cursor in the selected display may be made to blink on and off. By performing this emphasized display, the display selected and operated and the pointing cursor thereof can be simply distinguished from the other displays and the pointing cursors thereof.

The operation input unit 16 includes depth changing means. By instructing to change the depth through the use of the operation input unit 16 (for example, shifting a wheel 16S of the mouse 16 forward and backward or dragging the mouse 16 to the right and left), it is possible to change the depths of the pointing cursors 26T₁ and 26T₂ in the stereoscopic display 22.

The change in depth is performed specifically by changing the distance (the distance between the dotted lines) between the pointing cursor 26T₁ (the left-eye display) and the pointing cursor 26T₂ (the right-eye display) in FIG. 5. At least one of the pointing cursor 26L in the left-eye display 24L and the pointing cursor 26R in the right-eye display 24R moves to correspond to the change in distance between the pointing cursor 26T₁ and the pointing cursor 26T₂. The moving pointing cursor may be set to the pointing cursor 26T₁ or the pointing cursor 26T₂. Alternatively, depending on the positions of the pointing cursors 26T₁ and 26T₂ in the stereoscopic display 22, the pointing cursor 26T₂ may be made to move when the pointing cursors 26T₁ and 26T₂ are present in the left half of the stereoscopic display 22, the pointing cursor 26T₁ may be made to move when the pointing cursors 26T₁ and 26T₂ are present in the right half of the stereoscopic display 22, and the pointing cursor 26T₂ may be made to move when they are present at the center thereof.

When the distance between the pointing cursor 26T₁ (the left-eye display) and the pointing cursor 26T₂ (the right-eye display) in FIG. 5 is excessively large, the stereoscopic view is destroyed (the operator cannot recognize the stereoscopic view). Accordingly, when the distance between the right-eye display and the left-eye display is equal to or more than a predetermined distance, warning means that gives a warning may be provided. For example, the warning means may blink on and off the pointing cursor 26 or may change the color of the pointing cursor 26. Various warning means such as sound and pop-up on the display screen can be employed. An example of the predetermined distance is an average interocular distance of the operator or a human being, but the predetermined distance is not particularly limited.

The warning means may stop (limit) the movement of the pointing cursor 26T₁ and the pointing cursor 26T₂ so as not to be separated from each other by a predetermined distance, or may re-interlock the pointing cursor 26T₁ and the pointing cursor 26T₂ regardless of the change in depth so as not to separate the pointing cursor 26T₁ and the pointing cursor 26T₂ from each other by a predetermined distance or more.

The warning of the warning means is performed by the control unit 54 via the display control unit 56, when the distance between the pointing cursor 26T₁ and the pointing cursor 26T₂ is always calculated by the control unit 54 and the distance is equal to or more than a predetermined value.

As shown in FIG. 6, the pointing cursors 26 (26T₁, 26T₂, 26L, and 26R) include position designating means (markers) 28 (28T₁, 28T₂, 28L, and 28R). The markers 28 can be disposed at any position in the stereoscopic display and the planar display and are reflected in another display when they are disposed in any one display, similarly to the cursors 26. Similarly to the pointing cursors 26, the markers 28 include the depth changing means in the stereoscopic display, and may include the warning means so as to prevent the destruction of the stereoscopic view.

Similarly to the pointing cursors, the markers are calculated in position through the use of the control unit 54 of the console 30 of the stereoscopic display apparatus 10, are drawn through the use of the display control unit 56, and are displayed in the stereoscopic display, the right-eye display, and the right-eye display.

Although the case where the stereoscopic display 22 is selected by the display selecting means of the selection cursor 26 has hitherto been stated by FIGS. 4 to 6, the left-eye display 24L or the right-eye display 24R may be selected by the display selecting means of the pointing cursors 26.

An example thereof is a case where a position is designated by the use of the marker 28 in the stereoscopic display 22 but the position of the marker 28 in the planar display is not satisfactory and it is intended to directly finely adjust the positions of the markers 28 (the markers 28L and 28R) in the planar display.

It is often possible to simply and accurately perform adjustment by adjusting the marker positions while viewing the pointing cursor in the planar display.

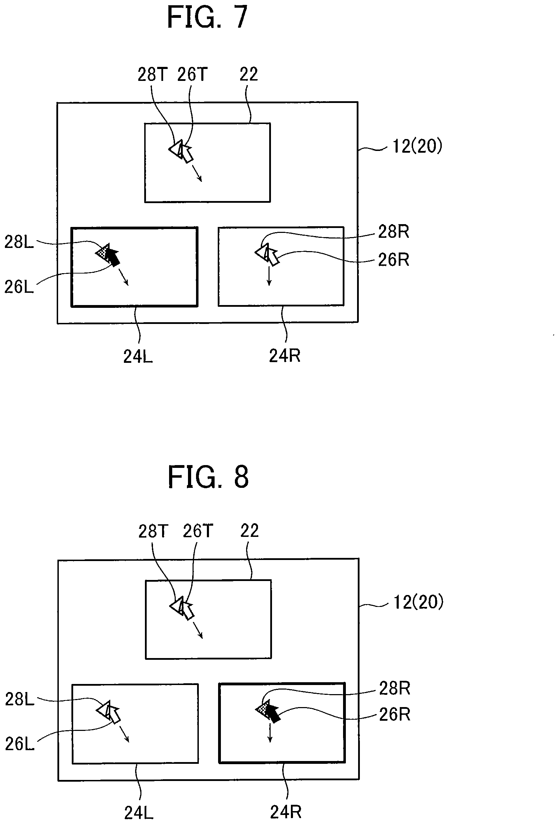

FIG. 7 is a diagram illustrating a case where the left-eye display 24L is selected by the display selecting means of the selection cursor 26 after the position is designated by the marker 28. For the purpose of convenient explanation, the same image as the left-eye display 24L is shown as a stereoscopic display in the stereoscopic display 22 but the stereoscopic display is recognized by the operator through the use of the pair of polarizing glasses.

As described above, the pointing cursor 26L and the frame of the left-eye display 24L are emphasized, whereby it can be seen that the left-eye display 24L is selected by the display selecting means.

As shown in FIG. 7, when the pointing cursor 26L is shifted in the left-eye display 24L, the pointing cursor 26R of the right-eye display 24R may be shifted, for example, by the vertical movement of the pointing cursor 26L.

In this case, the vertical movement of the pointing cursor 26L causes the vertical movement of the pointing cursor 26T in the stereoscopic display 22, but the horizontal movement of the pointing cursor 26L causes the movement in the depth direction of the pointing cursor 26T in the stereoscopic display 22.

The movement of the pointing cursor 26R in the right-eye display 24R is also similar. As shown in FIG. 8, the vertical movement of the pointing cursor 26R corresponds to the vertical movement of the pointing cursor 26T in the stereoscopic display 22, but the horizontal movement of the pointing cursor 26R corresponds to the movement in the depth direction of the pointing cursor 26T in the stereoscopic display 22.

When movement interlocking with the horizontal movement is intended, the operation input unit 16 may be operated to change the horizontal movement to simple movement from the adjustment in the depth direction.

The marker position information (the marker position information in the left-eye display 26L and the marker position information in the right-eye display 26R) after being adjusted can be stored in correlation with the stereoscopic display (the right-eye image and the left-eye image) and can be called out in response to an instruction from the operation input unit 16 if necessary.

When an image is printed out on a paper medium or the like, any one of the right-eye display and the left-eye display may be printed out, or the right-eye image and the left-eye image may be horizontally arranged and printed out so as to allow a stereoscopic view with naked eyes.

Hitherto, the configuration of an example of the stereoscopic display apparatus according to the present invention has been described.

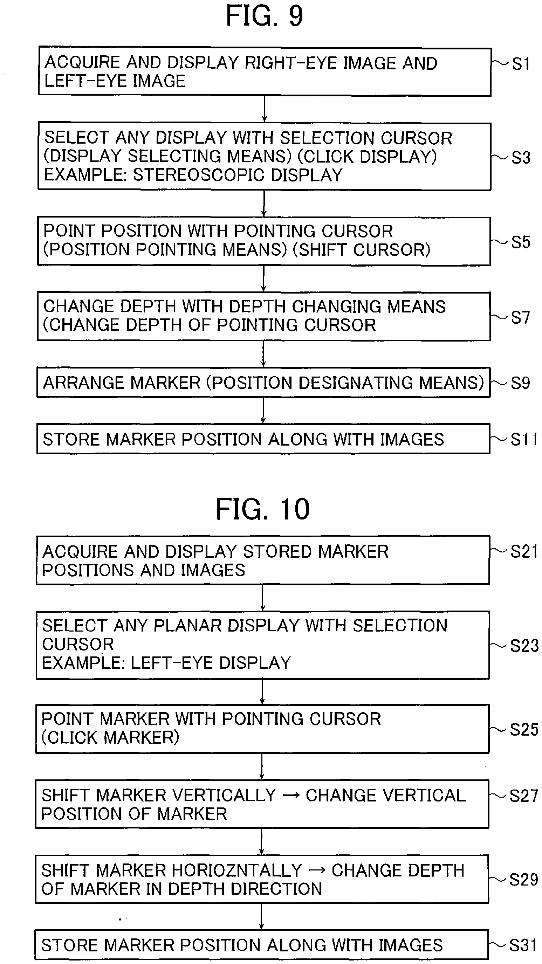

The operation of the stereoscopic display apparatus according to the present invention will be described below in brief with reference to the steps of the flowcharts shown in FIGS. 9 and 10. The flowchart shown in FIG. 9 relates to the arrangement of a marker in a stereoscopic display and the flowchart shown in FIG. 10 relates to fine adjustment of the marker position in the stereoscopic display through the use of the planar displays.

First, the arrangement of a marker in the stereoscopic display 22 will be described with reference to the steps of the flowchart shown in FIG. 9.

In step S1, the operation input unit (mouse) 16 of the stereoscopic display apparatus 10 is operated to acquire a right-eye image and a left-eye image necessary for a stereoscopic display from a radiographic imaging apparatus not shown or an image server 32 via the control unit 54 and the image acquiring unit 50 of the console 30,,a predetermined image process is performed by the image processing unit 52, a display process (a process for the first image display unit 18L) such as inversion of an image is performed by the display control unit 56, and the stereoscopic display, the left-eye display, and the right-eye display are displayed on the stereoscopic display unit 12 (S1).

In step S3, by operating the mouse 16, a display to be operated is selected (for example, the display is clicked) through the use of the display selecting means (selection cursor) (S3). Here, it is assumed that the stereoscopic display 22 is selected. The operation of the selection cursor 26 and the pointing cursors 26T₁, 26T₂, 26L, and 26R using the operation input unit 16 is reflected in the display screen via the control unit 54 and the display control unit 56. Various display processes on the display screen (that is, the first image display unit 18L and the second image display unit 18R) are performed by the control unit 54 and the display control unit 56.

When a display (here, the stereoscopic display 22) is selected, the pointing cursors 26L and 26R interlocking with the pointing cursors 26T in the selected display are displayed in the displays (here, the left-eye display 24L and the right-eye display 24R) other than the selected display, respectively. To distinguish the selected display from the other displays, the frame of the selected display may be emphasized or the pointing cursor in the selected display may be displayed to be distinguished from the pointing cursors in the other displays.

In step S5, the mouse 16 is operated in the selected stereoscopic display 22 to shift the pointing cursor and to point a predetermined position (S5).

When it is intended to change the pointing cursor 26T in the depth direction, the pointing cursor 26T may be changed in the depth direction by the use of the depth changing means (the wheel 16S of the mouse or the like) in step S7 (S7). The pointing cursor 26T is shifted to a predetermined position in the stereoscopic display 22 so as to include the depth direction. As described above, the calculation of the position in the depth direction of the pointing cursor 26T, that is, the calculation of the pointing cursors 26L and 26R in the right-eye image 24R and the left-eye image 24L, is performed by the control unit 54 of the console 30 and is reflected in the display screen through the use of the display control unit 56. The same is true of the position designating means (marker) 28 to be described later.

The position designating means (marker) 28T is then arranged in the stereoscopic display 22. In step S9, the marker 28T is arranged below the pointing cursor 26T shifted to the predetermined position in response to an instruction from the mouse 16 (S9). For example, the right side of the mouse 16 may be clicked and the marker 28T may be arranged using the displayed menu, or the marker 28T may be arranged by double click.

After arranging the marker 28T, in step S11, the position information of the marker 28T is stored in the storage unit 58 of the console 30 or the image server 32 along with the stereoscopic display (including the right-eye display and the left-eye display) (S11).

Hitherto, the operation of the stereoscopic display apparatus 10 when arranging the marker in the stereoscopic display has been described.

The adjustment of the marker position in the stereoscopic display will be described below with reference to the steps of the flowchart shown in FIG. 10.

In step S21, first, the right-eye image and the left-eye image necessary for a stereoscopic display stored along with the position information of the marker 28 are acquired from the storage unit 58 of the console 30 or the image server 32 by the stereoscopic display apparatus 10, and are displayed as the stereoscopic display 22, the left-eye display 24L, and the right-eye display 24R in which markers are arranged in advance on the stereoscopic display unit 12 (S21).

In step S23, by operating the mouse 16, any planar display is selected through the use of the selection cursor 26 (S23). Here, it is assumed that the left-eye display 24L is selected.

When the left-eye display 24L is selected through the use of the selection cursor 26, the frame of the left-eye display 24L is emphasized and the pointing cursors 26T and 26R interlocking with the pointing cursor 26L in the left-eye display 24L are displayed in the stereoscopic display 22 and the right-eye display 24R, respectively.

In step S25, the left-eye display 24L and a marker 28L is pointed (clicked) through the use of the pointing cursor 26L in the left-eye display 24L (S25). When the marker 28L is pointed, the arrangement of the pointed marker 28L can be changed.

In step S27, for example, when the marker 28L is vertically shifted in the left-eye display 24L, the marker 28T in the stereoscopic display 22 vertically moves (S27).

In step S29, for example, when the marker 24L is horizontally shifted, the position of the marker 28T in the stereoscopic display 22 is changed in the depth direction (S29).

By finely adjusting the marker position in the planar displays 24L and 24R, a desired position can be designated in the planar displays 24L and 24R and the stereoscopic display 22 through the use of the marker.

After finely adjusting the marker position, in step S31, the marker position information is stored in the storage unit 58 or the image server along with the stereoscopic display (including the right-eye image and the left-eye image) (S31).

Hitherto, the operation of the stereoscopic display apparatus 10 when the marker position in the stereoscopic display 22 is finely adjusted using the planar displays 24 has been described.

Although it has been stated in the above-mentioned embodiment that the number of position designating means (markers) is one for the purpose of convenient explanation, plural markers may be arranged and plural positions in the stereoscopic display and the planar displays may be designated. At this time, numerals or signs may be allocated to the centers of the markers for the purpose of identification.

When plural markers are present, the marker under the operation such as shift or change in depth may be emphasized as described above so as to be distinguished from the other markers not operated.

All of the stereoscopic display, the left-eye display, and the right-eye display may not be displayed always and one or more images may not be displayed if necessary. The arrangement of the displays may be appropriately changed.

Although not shown in the drawings, a character or a sign may be displayed under the stereoscopic display, the left-eye display, and the right-eye display so as to distinguish the displays from each other. For example, specific display names such as "stereoscopic display", "left-eye display", and "right-eye display" or signs such as "3D", "L", and "R" may be displayed. Accordingly, the operator can see the correspondence between the displays in the stereoscopic display unit 12 and these three displays. This display is performed through the use of the control unit 54 and the display control unit 56 as described above.

The display position can be preferably changed in accordance with the operator's instruction.

As described in the above-mentioned embodiment, when the stereoscopic display is located on the upside, the left-eye display is located on the lower-left side, and the right-eye display is located on the lower-right side, the actual right and left sides correspond to the right and left sides on the screen, which it is easy to understand. When wearing a pair of polarizing glasses, the operator can distinguish the shutting of the right eye and the shutting of the left eye.

Although it has been stated in the embodiment that the positions of the cursors or markers interlock with each other in the right-eye display and the left-eye display, this interlocking may be released in accordance with the operator's instruction.

Tomosynthesis imaging, CT imaging, or the like is assumed as the radiographic imaging. It is assumed in advance that the radiographic position (radiation source position) is shifted at one radiography time to capture plural radiographic images.

In general and simple radiography including mammography, when the radiographic position is shifted in a predetermined direction on the premise of a stereoscopic display and plural radiographic images are captured in advance, it is possible to perform a stereoscopic display using two images thereof.

While the stereoscopic display apparatus according to the present invention has been described in detail, the present invention is not limited to the embodiment, but may be improved or modified in various forms without departing from the concept of the present invention.

## Claims

1. A stereoscopic display apparatus that can perform a stereoscopic display using a right-eye image and a left-eye image acquired through radiography, comprising:
image display means that can display a planar image of at least one of the right-eye image and the left-eye image as a planar display along with the stereoscopic display;
display selecting means that selects any one of the stereoscopic display and the planar display; and
position pointing means that can be operated in the selected stereoscopic display or planar display and that can be operated to interlock in the stereoscopic display and the planar display,
wherein the position pointing means includes depth changing means that changes the depth thereof in the stereoscopic display.

2. The stereoscopic display apparatus according to claim 1, wherein when the depth of the position pointing means is changed, the position of the position pointing means in at least one of the right-eye display and the left-eye display is changed with the change of the depth.

3. The stereoscopic display apparatus according to claim 1 or 2, wherein when the position of the position pointing means in at least one of the right-eye display and the left-eye display is changed, the depth of the position pointing means in the stereoscopic display is changed.

4. The stereoscopic display apparatus according to any one of claims 1 to 3, wherein the position pointing means includes position designating means that can designate a position when disposed in the selected stereoscopic display or planar display and that is disposed to interlock in the stereoscopic display and the planar display,
wherein the depth changing means can change the depth of the position designating means.

5. The stereoscopic display apparatus according to claim 4, wherein when the depth of the position designating means is changed, the position of the position designating means in at least one of the right-eye display and the left-eye display is changed with the change of the depth.

6. The stereoscopic display apparatus according to claim 4 or 5, wherein when the arrangement of the position designating means in at least one of the right-eye display and the left-eye display is changed, the depth of the position designating means in the stereoscopic display is changed.

7. The stereoscopic display apparatus according to any one of claims 4 to 6, wherein a plurality of the position designating means can be arranged.

8. The stereoscopic display apparatus according to any one of claims 1 to 7, further comprising warning means that gives a warning when the position pointing means for a left eye and the position pointing means for a right eye in the stereoscopic display are separated from each other by a predetermined distance or more.

9. The stereoscopic display apparatus according to claim 8, wherein the warning includes stopping the changing of the distance between the position pointing means for the left eye and the position pointing means for the right eye, re-interlocking of the position pointing means for the left eye and the position pointing means for the right eye with each other, and displaying the purports of the stopping and the re-interlocking.

10. The stereoscopic display apparatus according to any one of claims 1 to 9, wherein the stereoscopic display and at least one of the right-eye display and the left-eye display are displayed on the single image display means.

11. The stereoscopic display apparatus according to any one of claims 1 to 9, wherein a plurality of the image display means are provided, and
wherein the stereoscopic display and at least one of the right-eye display and the left-eye display are displayed on the different image display means, respectively.
